(19) Europäisches Patentamt
European Patent Office
Office européen des brevets

(11) EP 4 134 864 A1

(12) **EUROPEAN PATENT APPLICATION**
published in accordance with Art. 153(4) EPC

(43) Date of publication:
**15.02.2023 Bulletin 2023/07**

(21) Application number: **20930380.9**

(22) Date of filing: **06.11.2020**

(51) International Patent Classification (IPC):
***G06F 40/117*** *(2020.01)*

(86) International application number:
**PCT/CN2020/127048**

(87) International publication number:
**WO 2021/203694 (14.10.2021 Gazette 2021/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.04.2020 CN 202010265146**

(71) Applicants:
- **BGI Genomics Co., Limited**
  **Shenzhen, Guangdong 518000 (CN)**
- **Bgi Health (HK) Company Limited**
  **Hong Kong 999077 (CN)**

(72) Inventors:
- **ZHOU, Jian**
  **Shenzhen, Guangdong 518000 (CN)**
- **KONG, Lingxiang**
  **Shenzhen, Guangdong 518000 (CN)**
- **YANG, Jiaobo**
  **Shenzhen, Guangdong 518000 (CN)**
- **HE, Zengquan**
  **Shenzhen, Guangdong 518000 (CN)**
- **WANG, Jin'an**
  **Shenzhen, Guangdong 518000 (CN)**

(74) Representative: **Schwarz & Partner Patentanwälte GmbH**
**Patentanwälte**
**Wipplingerstraße 30**
**1010 Wien (AT)**

(54) **HUMAN PHENOTYPIC STANDARD TERM DETERMINATION METHOD, AND RELATED DEVICE**

(57) A human phenotypic standard term determination method, and a related device. The method comprises: obtaining symptom description information (S100); performing query matching on word segmentation groups corresponding to various human phenotypic standard terms in a preset human phenotypic standard term library and the symptom description information, and determining the degree of matching between each human phenotypic standard term and the symptom description information (S200), wherein the word segmentation group comprises at least one segmented word list obtained by means of performing, according to a pre-set word segmentation rule, word segmentation on human phenotypic standard terms corresponding to the word segmentation group, and the segmented word list comprises at least one segmented word; and determining a human phenotypic standard term, the degree of matching of which meets a preset matching success condition (S300). According to the method, query matching is performed on symptom description information and word segmentation groups corresponding to various human phenotypic standard terms, and a human phenotypic standard term conforming to the symptom description information is screened out, thereby facilitating research and communication of the symptom description information by a technician, and also facilitating data analysis and mining of symptoms on the symptom description information.

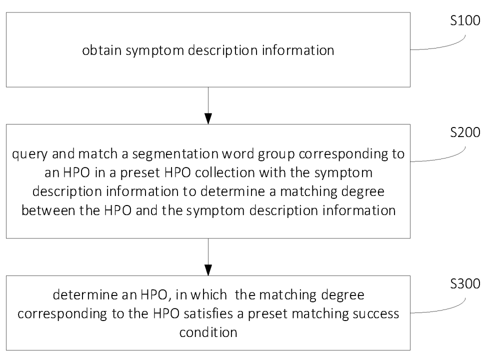


FIG. 1

EP 4 134 864 A1

## Description

This application claims priority to Chinese Patent Application No. 202010265146.4, filed on April 7, 2020, in China National Intellectual Property Administration, titled "Human Phenotypic Standard Term Determination Method, and Related Device", the entire content of which is incorporated herein by reference.

## TECHNICAL FIELD

The disclosure relates to a field of data processing technologies, and more particularly to a method for determining Human Phenotype Ontology and related devices.

## BACKGROUND

Human Phenotype Ontology (HPO) is a standard word group describing abnormal phenotype caused by human diseases. In order to facilitate unification of phenotype description expressions used by most of the domestic medical and scientific research workers, the Chinese Human Phenotype Ontology Consortium was established in China to translate HPO expressed in English into Chinese Human Phenotype Ontology (CHPO).

In actual situations, different doctors use various descriptions to describe symptoms corresponding to the same HPO in the symptom description information, which brings inconvenience to following clinical research and communication of the symptom, and makes it difficult for data analysis and data mining of the symptom.

## SUMMARY

In view of the above problem, the disclosure provides a method for determining HPO and related devices, to solve the above problem or at least partially solve the above problem. The related technical solution is as follows.

The method for determining a HPO includes:

obtaining symptom description information;

for a segmentation word list in a segmentation word group corresponding to an HPO in a preset HPO collection, querying, from the symptom description information, a segmentation word of the segmentation word list, determining a segmentation word included in both the segmentation word list and the symptom description information as a common segmentation word; determining the number of common segmentation words in the segmentation word list and respective positions of the common segmentation words in the symptom description information; determining a first similarity between the segmentation word list and the symptom description information based on the number of common segmentation words in the segmentation word list and the number of segmentation words in the segmentation word list; determining a measures of dispersion between the HPO and the symptom description information based on the respective positions of the common segmentation words in the symptom description information and respective positions of the common segmentation words in the HPO;

determining a second similarity between the HPO and the symptom description information based on a corresponding first similarity of each segmentation word list;

determining a matching degree between the HPO and the symptom description information based on the second similarity, the measures of dispersion, and the number of segmentation word lists in the segmentation word group; and determining an HPO, in which the matching degree corresponding to the HPO satisfies a preset matching success condition.

Optionally, segmentation words are different between each segmentation word list of the segmentation word group, and the number of words of each segmentation word is the same for the same segmentation word list.

Optionally, after determining the HPO, in which the matching degree corresponding to the HPO satisfies the preset matching success condition, the method further includes:

determining, from the symptom description information, a description expression corresponding to the HPO; and detecting whether an adjacent expression adjacent to the description expression is a preset negative expression, and labeling the adjacent expression in response to determining that the adjacent expression is the preset negative expression.

Optionally, after determining the HPO, in which the matching degree corresponding to the HPO satisfies the preset matching success condition, the method further includes:

determining, from the symptom description information, a description expression corresponding to the HPO; and determining whether the description expression is consistent with the HPO, determining that the HPO is completely matched with the description expression, in response to the description expression being consistent with the HPO, and determining that the HPO is roughly matched with the description expression, in response to the description expression being inconsistent with the HPO.

Optionally, determining the second similarity between the HPO and the symptom description information based on the corresponding first similarity of each segmentation word list includes:

determining a third similarity between the HPO and the symptom description information based on the corresponding first similarity of each segmentation word list; and determining the second similarity between the HPO and the symptom description information by multiplying the third similarity by an adjustable weighting factor.

Optionally, determining the measures of dispersion between the HPO and the symptom description information based on respective positions of the common segmentation words in the symptom description information and the respective positions of the common segmentation words in the HPO, includes:

determining a first position vector of the segmentation word list in the symptom description information based on the respective positions of the common segmentation words in the symptom description information; determining a second position vector of the segmentation word list in the HPO based on the respective positions of the common segmentation words in the HPO; and determining the measures of dispersion between the HPO and the symptom description information based on a corresponding first position vector and a corresponding second position vector of each segmentation word list.

Optionally, determining the matching degree between the HPO and the symptom description information based on the second similarity, the measures of dispersion, and the number of segmentation word lists in the segmentation word group, includes:

$$AEEV=\frac{VAR}{N}\times Score$$

where, *AEEV* is the matching degree between the HPO and the symptom description information, *VAR* is the measures of dispersion between the HPO and the symptom description information, and *Score* is the second similarity between the HPO and the symptom description information, and *N* is the number of segmentation word lists in the segmentation word group;

$$Score=\gamma\times exp(preScore)$$

where, $\gamma$ is an adjustable weighting factor, and *preScore* is a third similarity,

$$preScore=\sum_{n=1}^{N}\alpha\beta_n w_n \log p_n$$

where, $n$ is a serial number of each segmentation word list, $\alpha\beta_n w_n \log p_n$ is the first similarity of the segmentation word list $n$, where $1\leq n\leq N$, $\alpha$ is an adjustment factor, $\beta_n$ is a normalization factor corresponding to the segmentation word list $n$, $w_n$ is a weight corresponding to the segmentation word list $n$, and $p_n$ is a ratio of the number of common segmentation words in the segmentation word list $n$ to the number of segmentation words in the segmentation word list $n$;

$$VAR=\sqrt{\frac{\sum_{n=1}^{N}\left(a_n-b_n-\frac{\sum_{n=1}^{N}(a_n-b_n)}{N}\right)^2}{N-1}}$$

where, $a_n$ is a first position vector of the segmentation word list $n$ in the symptom description information, and $b_n$ is a second position vector of the segmentation word list $n$ in the HPO.

An apparatus for determining a HPO includes: a symptom description information obtaining unit, a matching degree determining unit, and a HPO determining unit. The matching degree determining unit includes: a common segmentation word determining sub-unit, a common segmentation word number determining sub-unit, a common segmentation word position determining sub-unit, a first similarity determining sub-unit, a measures of dispersion determining sub-unit, a second similarity determining sub-unit, and a matching degree determining sub-unit.

The symptom description information obtaining unit is configured to obtain symptom description information.

The common segmentation word determining sub-unit is configured to, for a segmentation word list in a segmentation word group corresponding to an HPO in a preset HPO collection, query, from the symptom description information, a segmentation word of the segmentation word list, and determine a segmentation word included in both the segmentation word list and the symptom description information as a common segmentation word.

The common segmentation word number determining sub-unit is configured to determine the number of common segmentation words in the segmentation word list.

The common segmentation word position determining sub-unit is configured to determine respective positions of the common segmentation words in the symptom description information.

The first similarity determining sub-unit is configured to determine a first similarity between the segmentation word list and the symptom description information based on the number of common segmentation words in the segmentation word list and the number of segmentation words in the segmentation word list.

The measures of dispersion determining sub-unit is configured to determine a measures of dispersion between the HPO and the symptom description information based on the respective positions of the common segmentation words in the symptom description information and respective positions of the common segmentation words in the HPO.

The second similarity determining sub-unit is configured to determine a second similarity between the HPO and the symptom description information based on a corresponding first similarity of each segmentation word list.

The matching degree determining sub-unit is configured to determine a matching degree between the HPO and the symptom description information based on the second similarity, the measures of dispersion, and the number of segmentation word lists in the segmentation word group.

The HPO determining unit is configured to determine an HPO, in which the matching degree corresponding to the HPO satisfies a preset matching success condition.

Optionally, the apparatus further includes: a description expression determining unit, a negative expression detecting unit, and a negative expression labeling unit.

The description expression determining unit is configured to determine, from the symptom description information, a description expression corresponding to the HPO, after determining the HPO, in which the matching degree corresponding to the HPO satisfies the preset matching success condition.

The negative expression detecting unit is configured to detect whether an adjacent expression adjacent to the description expression is a preset negative expression, and trigger the negative expression labeling unit in response to detecting that the adjacent expression is the preset negative expression.

The negative expression labeling unit is configured to label the adjacent expression.

Optionally, the apparatus further includes: a description expression determining unit, an expression determining unit and an expression matching determining unit.

The description expression determining unit is configured to determine, from the symptom description information, a description expression corresponding to the HPO.

The expression determining unit is configured to determine whether the description expression is consistent with the HPO, trigger the expression matching determining unit in response to the description expression being consistent with the HPO, in which the expression matching determining unit is configured to determine that the HPO is completely matched with the description expression, and trigger the expression matching determining unit in response to the description expression being inconsistent with the HPO, in which the expression matching determining unit is configured to determine that the HPO is roughly matched with the description expression.

According to the above technical solution, the disclosure provides a method for determining a HPO and related devices. Symptom description information is obtained. For a segmentation word list in a segmentation word group corresponding to an HPO in a preset HPO collection, a segmentation word of the segmentation word list is queried from the symptom description information, and the matching degree between the HPO and the symptom description information is determined. The segmentation word group includes at least one segmentation word list obtained by means of performing, according to a pre-set word segmentation rule, word segmentation on the HPO corresponding to the segmentation word group, and the segmentation word list includes at least one segmentation word. A HPO is determined, the matching degree corresponding to the HPO satisfies a preset matching success condition. According to the method, querying and matching is performed on the symptom description information and segmentation word groups corresponding to various HPOs, and the HPOs conforming to the symptom description information are screened out, thereby

facilitating research and communication of the symptom description information by a technician, and facilitating data analysis and data mining of symptoms on the symptom description information.

The above description is only an overview of the technical solution of the disclosure. In order to clearly understand the technical means of the disclosure, the technical means can be implemented in accordance with the content of the specification. In order to make the above and additional purposes, features and advantages of the disclosure more obvious and understandable, the specific implementations of the disclosure are described below.

## BRIEF DESCRIPTION OF THE DRAWINGS

In order to clearly illustrate technical solution of embodiments of the disclosure, a brief description of drawings used in embodiments is given below. Obviously, the drawings in the following descriptions are only part of embodiments of the disclosure, and for those skilled in the art, other drawings can be obtained according to these drawings without inventive works.

FIG. 1 is a flowchart illustrating a method for determining a Human Phenotype Ontology (HPO) according to embodiments of the disclosure.

FIG. 2 is a flowchart illustrating another method for determining a HPO according to embodiments of the disclosure.

FIG. 3 is a flowchart illustrating another method for determining a HPO according to an embodiment of the disclosure.

FIG. 4 is a flowchart illustrating another method for determining a HPO according to embodiments of the disclosure.

FIG. 5 is a schematic diagram illustrating an output result of HPO according to embodiments of the disclosure.

FIG. 6 is a flowchart illustrating another method for determining a HPO according to embodiments of the disclosure.

FIG. 7 is a flowchart illustrating another method for determining a HPO according to embodiments of the disclosure.

FIG. 8 is a schematic diagram illustrating an output result of HPO according to embodiments of the disclosure.

FIG. 9 is a schematic diagram illustrating an apparatus for determining a HPO according to embodiments of the disclosure.

## DETAILED DESCRIPTION

Although the drawings show exemplary embodiments of the disclosure, it should be understood that the disclosure can be implemented in various forms and should not be limited by the embodiments set forth herein. Instead, these embodiments are provided to enable a more thorough understanding of the disclosure and to fully convey the scope of the disclosure to those skilled in the art.

As illustrated in FIG. 1, embodiments of the disclosure provide a method for determining a HPO. The method includes the following.

In S100, symptom description information is obtained.

The symptom description information may be a record of medical activities such as examination and diagnosis of patient's diseases by medical staff. The symptom description information can be a sentence or a paragraph composed of multiple words or phrases. Embodiments of the disclosure may provide an interface for a user to input the symptom description information, and the user can edit and input the symptom description information on the interface or upload a file edited with the symptom description information. In embodiments of the disclosure, a text scanning device may also be used to obtain the symptom description information handwritten by the medical staff through scanning. Certainly, the disclosure can also obtain the symptom description information that the medical staff has entered in an existing diagnosis and treatment system from the diagnosis and treatment system (for example, the diagnosis and treatment system installed on computers of various departments of a hospital).

In S200, a segmentation word group corresponding to the HPO in a preset HPO collection is queried and matched with the symptom description information, and a matching degree between the HPO and the symptom description information is determined. The segmentation word group includes: at least one segmentation word list obtained after word segmentation is performed on the HPO corresponding to the segmentation word group according to a preset word segmentation rule, and the segmentation word list includes at least one segmentation word.

The preset HPO collection may be a database that stores various HPOs. Corresponding Chinese and English

vocabulary of various HPOs can be included in the database. For example, CHPO: "肠扭转" corresponds to HPO: "Volvulus". The preset word segmentation rule can be a rule for the technicians to reassemble the HPOs into a word sequence according to a certain regulation. Optionally, the preset word segmentation rule may be an algorithm based on a statistical language model (SLM), or may be a word segmentation rule customized by the technicians. For example, in embodiments of the disclosure, the HPO can be divided based on an *n-gram* algorithm of the SLM. In detail, embodiments of the disclosure can divide the HPO through the n-gram algorithm based on different numbers of characters to obtain multiple segmentation words of different numbers of characters. For example, assuming that one Chinese character is one character, in embodiment of the disclosure, "肠扭转" ("volvulus" in Chinese) can be divided according to the number of characters equaling to 1, the number of characters equaling to 2 or the number of characters equaling to 3. The segmentation word list obtained by performing the word segmentation according to the number of characters equaling to 1 includes "肠", "扭" and "转", the segmentation word list obtained by performing the word segmentation according to the number of characters equaling to 2 includes "肠扭" and "扭转", and the segmentation word list obtained by performing the word segmentation according to the number of characters equaling to 3 includes "肠扭转".

Optionally, the segmentation words are different between each segmentation word list of the segmentation word group, and the number of words of each segmentation word is the same for the same segmentation word list.

In detail, in embodiments of the disclosure, the segmentation words in the word segmentation result may be classified according to a certain rule to obtain the segmentation word lists. For example, in embodiments of the disclosure, the segmentation words in the word segmentation result can be classified based on the number of characters. For the word segmentation result of "肠扭转" in the foregoing example, the segmentation words having the number of characters equaling to 1, i.e., "肠", "扭" and "转", are classified as the segmentation word list A, the segmentation words having the number of characters equaling to 2, i.e., "肠扭" and "扭转", are classified as the segmentation word list B, and the segmentation word having the number of characters equaling to 3, i.e., "肠扭转", is classified as the segmentation word list C.

It is understandable that the preset word segmentation rule can be a word segmentation rule used by existing word segmentation tools. The existing word segmentation tools may include: jieba word segmentation, language technology platform (LTP) and natural language processing & information retrieval sharing platform (NLPIR).

It should be noted that the above examples are only used to illustrate the vocabulary when the HPO are expressed in Chinese. It is understandable that embodiments of the disclosure can perform word segmentation on HPO in language expressions including English.

Optionally, based on the method in FIG. 1, as illustrated in FIG. 2, embodiments of the disclosure provide another method for determining a HPO, and the S200 may include the following.

In S210, for any one of segmentation word lists in a segmentation word group corresponding to each HPO, segmentation words of the segmentation word list are queried from the symptom description information. A segmentation word included in both the segmentation word list and the symptom description information is determined as a common segmentation word. In S211, the number of common segmentation words in the segmentation word list is determined and respective positions of the common segmentation words in the symptom description information are determined. In S212, a first similarity between the segmentation word list and the symptom description information is determined based on the number of common segmentation words in the segmentation word list and the number of segmentation words in the segmentation word list. In S213, a measures of dispersion between the HPO and the symptom description information is determined based on the respective positions of the common segmentation words in the symptom description information and respective positions of the common segmentation words in the HPO.

In order to facilitate understanding of the common segmentation words in S210, an example is used for explanation. When the symptom description information is "因肠旋转不良后死亡 (Chinese characters and the meaning in English is "dead due to volvulus")", segmentation words "肠", "扭" and "转" in the segmentation word list are queried in the symptom description information. Since the segmentation words "肠" and "转" are included in both the segmentation word list and the symptom description information, the segmentation words "肠" and "转" and determined as the common segmentation words. Certainly, if the segmentation word in the segmentation word list is "肠 扭 转", there is no common segmentation word between the segmentation word list and the symptom description information.

The S212 can include: determining a first similarity between the segmentation word list and the symptom description information based on a ratio of the number of the common segmentation words in the segmentation word

list to the number of the segmentation words in the segmentation word list.

For example, if a segmentation word list includes three segmentation words "肠", "扭" and "转"and the segmentation words "肠" and "转" are the common segmentation words between the segmentation word list and the symptom description information, the first similarity between the segmentation word list and the symptom description information is 2/3.

Optionally, based on FIG. 2, as illustrated in FIG. 3, embodiments of the disclosure provide another method for determining an HPO. The S213 includes the following.

In S213a, a first position vector of the segmentation word list in the symptom description information is determined based on respective positions of the common segmentation words in the symptom description information.

The position of a common segmentation word in the symptom description information can be a character serial number. It should be noted that the serial number can be related to the number of characters in each segmentation word in the segmentation word list. For example, when the number of characters of each segmentation word in a segmentation word list is 1, a character serial number in the symptom description information can represent one character. When the number of characters of each segmentation word in a segmentation word list is 2, a character serial number in the symptom description information can represent two characters. For ease of understanding, an example is used here for further illustration, when the symptom description information is "长 骨部分弯曲", if the common segmentation words are "长" and "弯", the character serial number of the common segmentation word "长" in the symptom description information can be "1", and the character serial number of the common segmentation word "弯" in the symptom description information can be "5". If the common segmentation word is "弯 曲", the character serial number of the common segmentation word "弯 曲" in the symptom description information can be "5".

The first position vector may be a one dimensional list composed of the character serial numbers of the common segmentation words in the symptom description information. For ease of understanding, here is an example for illustration, when the symptom description information is "疑似患者，孕 32 周+5，胎儿骨干发育不良如孕 26 周，长骨部分弯曲。有家族遗传病史", if the common segmentation words between a segmentation word list and the symptom description information are "长", "骨", "弯" and "曲", the first position vector of the segmentation word list in the symptom description information is [27, 28, 31, 32]. If the common segmentation words between a certain segmentation word list and the symptom description information are "长骨", "骨 弯" and "弯 曲", the second position vector of the segmentation word list in the HPO is [1, 2, 3].

In S213b, a second position vector of the segmentation word list in the HPO is determined based on the respective positions of the common segmentation words in the HPO.

The position of the common segmentation word in the HPO can be a character serial number. It should be noted that the character serial number can be related to the number of characters in each segmentation word in the word segmentation list. For example, when the number of characters of each segmentation word in the segmentation word list is 1, one character serial number in the HPO can represent a character. When the number of characters in each segmentation word in the segmentation word list is 2, one character serial number in the HPO can represent two characters. For ease of understanding, an example is used for further illustration. When the HPO is "长骨弯曲", if the common segmentation words are "长" and "弯", the character serial number of the common segmentation word "长" in the HPO can be "1", and the character serial number of the common segmentation word "弯" in the HPO can be "3". If the common segmentation word is "弯 曲", the character serial number of the common segmentation word "弯 曲" in the HPO can be "3". It should be noted that a punctuation mark also occupies one character serial number. The second position vector may be a one dimensional list consisting of the character serial numbers of the common segmentation words in the HPO. For ease of understanding, an example is used here for illustration. When the HPO is "长骨弯曲", if the common segmentation words between a certain segmentation word list and the symptom description information are "长", "骨", "弯" and "曲", the second position vector of the segmentation word list in the HPO is [1, 2, 3, 4]. If the common segmentation words between a certain segmentation word list and the symptom description infor-

mation are "长骨", "骨弯" and "弯曲", the second position vector of the segmentation word list in the HPO is [1, 2, 3].

In S213c, the measures of dispersion between the HPO and the symptom description information is determined based on a corresponding first position vector and a corresponding second position vector of each segmentation word list.

Optionally, the S213c includes:

$$VAR = \sqrt{\frac{\sum_{n=1}^{N}\left(a_n - b_n - \frac{\sum_{n=1}^{N}(a_n - b_n)}{N}\right)^2}{N-1}}$$

where, *VAR* is the measures of dispersion between the HPO and the symptom description information, *N* is the number of segmentation word lists in the segmentation word group, $a_n$ is a first position vector of the segmentation word list *n* in the symptom description information, and $b_n$ is a second position vector of the segmentation word list *n* in the HPO.

In S220, a second similarity between the HPO and the symptom description information is determined based on a corresponding first similarity of each segmentation word list.

Optionally, based on the method illustrated in FIG. 2, as illustrated in FIG. 4, embodiments of the disclosure provide another method for determining a HPO. The S220 includes the following.

In S221, a third similarity between the HPO and the symptom description information is determined based on the corresponding first similarity of each segmentation word list.

In S222, the second similarity between the HPO and the symptom description information is determined by multiplying the third similarity by an adjustable weighting factor.

Optionally, the S221 may include:

$$preScore = \sum_{n=1}^{N} \alpha\beta_n w_n \, logp_n$$

where, *n* is a serial number of each segmentation word list, *preScore* is the third similarity, $\alpha\beta_n w_n logp_n$ is the first similarity of the segmentation word list n, where $1 \leq n \leq N$, *N* is the number of segmentation words in the segmentation word list, $\alpha$ is an adjustment factor, $\beta_n$ is a normalization factor corresponding to the segmentation word list *n,* $w_n$ is a weight corresponding to the segmentation word list *n,* and $p_n$ is a ratio of the number of common segmentation words in the segmentation word list *n* to the number of segmentation words in the segmentation word list n.

The adjustable weighting factor can be set by the technicians. The technicians can adjust the adjustable weighting factor according to the execution result after each execution of the method for determining the HPO according to embodiments of the disclosure.

The normalization factor corresponding to the segmentation word list n can be expressed as:

$$\beta_n = \frac{logp_n - logp}{S^2}$$

where, *logp* is an average value of data $logp_1, \ldots, logp_n$, which can be expressed as:

$$logp = \frac{\sum_{n=1}^{N} logp_n}{n}$$

where, $S^2$ is a variance of data $logp_1, \ldots, logp_n$, which can be expressed as:

$$S^2 = \frac{\sum_{n=1}^{N}(logp_n - logp)^2}{n}$$

The weight corresponding to each segmentation word list can be set by the technicians. The technicians can adjust the weight according to the execution result after each execution of the method for determining the HPO according

to embodiments of the disclosure.

The S222 may include:

$$Score=\gamma \times exp(preScore)$$

where, *Score* is the second similarity between the HPO and the symptom description information, $\gamma$ is the adjustable weighting factor.

It is noted that the adjustable weighting factor is related to the HPO. In detail, embodiments of the disclosure may determine the adjustable weighting factor based on historical matching data of the HPO. In detail, in embodiments of the disclosure, the historical matching data of the HPO can be extracted from a historical matching database. The historical matching data may include: a first number of times the HPO is correctly matched, a second number of times the HPO is incorrectly matched, and a third number of times the HPO is manually labeled. Embodiments of the disclosure may perform a weighted sum of the first number, the second number and the third number to determine the adjustable weighting factor. According to the historical matching data of the HPO, embodiments of the disclosure determine the adjustable weighting factor used to calculate the matching degree between the HPO and the symptom description information, which can improve the probability that the HPO is correctly matched.

In S230, the matching degree between the HPO and the symptom description information is determined based on the second similarity, the measures of dispersion, and the number of segmentation word lists in the segmentation word group.

In order to facilitate the understanding of the matching degree between the HPO and the symptom description information, optionally, embodiments of the disclosure provide another method for determining the HPO. The S230 may include:

$$AEEV=\frac{VAR}{N}\times Score$$

where, *AEEV* is the matching degree between the HPO and the symptom description information, *VAR* is the measures of dispersion between the HPO and the symptom description information, *Score* is the second similarity between the HPO and the symptom description information, and *N* is the number of segmentation word lists in the segmentation word group;

$$Score=\gamma \times exp(preScore)$$

where, $\gamma$ is the adjustable weighting factor, and *preScore* is the third similarity,

$$preScore=\sum_{n=1}^{N}\alpha\beta_n w_n\, logp_n$$

where, *n* is a serial number of each segmentation word list, $\alpha\beta_n w_n logp_n$ is the first similarity of the segmentation word list *n*, where $1 \le n \le N$, $\alpha$ is an adjustment factor, $\beta_n$ is a normalization factor corresponding to the segmentation word list *n*, $w_n$ is a weight corresponding to the segmentation word list *n*, and $p_n$ is a ratio of the number of common segmentation words in the segmentation word list *n* to the number of segmentation words in the segmentation word list *n;*

$$VAR=\sqrt{\frac{\sum_{n=1}^{N}\left(a_n-b_n-\frac{\sum_{n=1}^{N}(a_n-b_n)}{N}\right)^2}{N-1}}$$

where, a, is a first position vector of the segmentation word list *n* in the symptom description information, and $b_n$ is a second position vector of the segmentation word list *n* in the HPO.

In S300, an HPO is determined, the matching degree corresponding to the HPO satisfies a preset matching success condition.

The preset matching success condition may be the matching degree between the HPO and the symptom description information is greater than a preset matching degree threshold. For ease of understanding, an example is used for illustration. If the matching degree between the HPO M and the symptom description information *T* is 9 and the matching degree between the HPO *N* and the symptom description information *T* is 4, when the preset matching degree threshold is 8, the HPO *M* is the HPO that satisfies the preset matching success condition.

In embodiments of the disclosure, an HPO whose matching degree does not satisfy the preset matching success condition is filtered out, and only the HPOs whose matching degree satisfies the preset matching success condition are output. It is understandable that, as illustrated in FIG. 5, an output result of HPO is provided by embodiments of the disclosure. The output result may include corresponding Chinese and English vocabulary of each of the HPOs whose matching degree satisfy the preset matching success condition. That is, embodiments of the disclosure can output the *CHPOs* and *HPOs* of the HPO at the same time, and when outputting the *HPO*, the serial number corresponding to the *HPO* can also be output at the same time. It is understandable that, in actual situations, there may be multiple HPOs whose matching degree with respect to the same symptom description information satisfies the preset matching success condition. As illustrated in FIG. 5, embodiments of the disclosure can output the HPOs simultaneously, and use a preset corresponding identifier (such as an arrow) to label the corresponding description expressions of the HPOs in the symptom description information, and output the description expressions.

With the method for determining a HPO according to embodiments of the disclosure, the symptom description information can be obtained. The segmentation word group corresponding to the HPO in the preset HPO collection is queried from the symptom description information, and the matching degree between the HPO and the symptom description information is determined. The segmentation word group includes at least one segmentation word list obtained by performing, according to a preset word segmentation rule, word segmentation on the HPO corresponding to the segmentation word group, and the segmentation word list includes at least one segmentation word. A HPO is determined, where the matching degree corresponding to the HPO satisfies the preset matching success condition. According to the method, querying and matching are performed on the symptom description information and segmentation word groups corresponding to various HPOs to determine the matching degree between each HPO and the symptom description information, and HPOs conforming to the symptom description information are determined, thereby facilitating research and communication of the symptom description information by a technician, and facilitating data analysis and data mining of symptoms on the symptom description information.

Optionally, FIG. 6 is a flowchart illustrating another method for determining a HPO according to embodiments of the disclosure. After the S300, the method further includes the following.

In S400, a description expression corresponding to the HPO is determined from the symptom description information.

In detail, in embodiments of the disclosure, according to the measures of dispersion of a common segmentation word in each segmentation word list corresponding to the HPO in the symptom description information, the vocabulary at this position is determined as the corresponding description expression of the HPO in the symptom description information. For example, the symptom description information is "该患者患有肠胃炎，并伴有肠旋转", the common segmentation words between the HPO "肠 扭 转" and the symptom description information are "肠" and "转". Although there is a common word segmentation "肠" in both "肠 胃 炎" and "肠旋转", the "肠旋转" also has the common segmentation word "转", and thus the "肠旋转" has a lower measures of dispersion of the common segmentation word compared to "肠

胃 炎". Therefore, the description expression in the symptom description information corresponding to the HPO "肠扭转" is "肠旋转".

In S500, it is detected whether an adjacent expression adjacent to the description expression is a preset negative expression, and the adjacent expression is labeled in response to determining that the adjacent expression is the preset negative expression.

The preset negative expressions can include "无", "未", "没", "不", "非", "别", "勿", "缺" and "1i". Embodiments of the disclosure can detect whether the adjacent expression adjacent to the description expression and before the description expression is a preset negative expression. When the adjacent expression is a preset negative expression, embodiments of the disclosure may label the adjacent expression with the preset negative identifier. For example, in embodiments of the disclosure, a font of the adjacent expression may be bolded, a font color of the adjacent expression may be changed, and a special symbol may be used to indicate the adj acent expression. By labeling the adjacent expression i.e., the preset negative expression in embodiments of the disclosure, it is convenient for the user to clearly

distinguish whether the description expressions in the symptom description information are intended to express a positive meaning or a negative meaning. In embodiments of the disclosure, when it is detected that the adjacent expression adjacent to the description expression is not a preset negative expression, the adjacent expression may not be labeled.

Optionally, FIG. 7 is a flowchart illustrating another method for determining a HPO according to embodiments of the disclosure. After the S300, the method further includes the following.

In S600, a description expression corresponding to the HPO is determined from the symptom description information.

The description of the S600 can refer to the above description of the S400, which will not be repeated here.

In S700, it is determined whether the description expression is consistent with the HPO. If the description expression is consistent with the HPO, the S800 is executed. If the description expression is inconsistent with the HPO, the S900 is executed.

In S800, it is determined that the HPO is completely matched with the description expression.

In S900, it is determined that the HPO is roughly matched with the description expression.

Based on the output result illustrated in FIG. 5, FIG. 8 is a schematic diagram illustrating an output result of a HPO according to embodiments of the disclosure. Embodiments of the disclosure may output the HPO whose matching degree satisfies the preset matching success condition, and output the matching mode of the HPO and the description expression as completely matched or roughly matched.

Corresponding to the foregoing method embodiments, embodiments of the disclosure also provide an apparatus for determining a HPO. The structure of the apparatus is illustrated in FIG. 9 and the apparatus may include: a symptom description information obtaining unit 100, a matching degree determining unit 200, and a HPO determining unit 300.

The symptom description information obtaining unit 100 is configured to obtain symptom description information.

The symptom description information may be a record of medical activities such as examination and diagnosis of patient's diseases by medical staff. The symptom description information can be a sentence or a paragraph composed of multiple words or phrases. Embodiments of the disclosure may provide an interface for a user to input the symptom description information, and the user can edit and input the symptom description information on the interface or upload a file edited with the symptom description information. In embodiments of the disclosure, a text scanning device may also be used to obtain the symptom description information handwritten by medical staff through scanning. Certainly, the disclosure can also obtain the symptom description information that the medical staff has entered in an existing diagnosis and treatment system from the diagnosis and treatment system (for example, the diagnosis and treatment system installed on computers of various departments of a hospital).

The matching degree determining unit 200 is configured to query and match a segmentation word group corresponding to the HPO in a preset HPO collection with the symptom description information, and determine a matching degree between the HPO and the symptom description information. The segmentation word group includes: at least one segmentation word list obtained after word segmentation is performed on the HPO corresponding to the segmentation word group according to a preset word segmentation rule, and the segmentation word list includes at least one segmentation word.

The preset HPO collection may be a database that stores various HPOs. Corresponding Chinese and English vocabulary of various HPOs can be included in the database. Optionally, the preset word segmentation rule may be an algorithm based on a statistical language model (SLM), or may be a word segmentation rule customized by the technicians.

Optionally, the segmentation words are different between each segmentation word list of the segmentation word group, and the number of words of each segmentation word is the same for the same segmentation word list.

In detail, in embodiments of the disclosure, the segmentation words in the word segmentation result can be classified according to a certain rule to obtain the segmentation word lists.

It is understandable that the preset word segmentation rule can be a word segmentation rule used by existing word segmentation tools. The existing word segmentation tools may include: jieba word segmentation, language technology platform (LTP) and natural language processing & information retrieval sharing platform (NLPIR).

Optionally, embodiments of the disclosure also provide an apparatus for determining a HPO. The matching degree determining unit 200 includes: a common segmentation word determining sub-unit, a common segmentation word number determining sub-unit, a common segmentation word position determining sub-unit, a first similarity determining sub-unit, a measures of dispersion determining sub-unit, a second similarity determining sub-unit, and a matching degree determining sub-unit.

The common segmentation word determining sub-unit is configured to, for any one of segmentation word lists in a segmentation word group corresponding to each HPO, query, from the symptom description information, segmentation words of the segmentation word list, and determine a segmentation word included in both the segmentation word list and the symptom description information as a common segmentation word.

The common segmentation word number determining sub-unit is configured to determine the number of common segmentation words in the segmentation word list.

The common segmentation word position determining sub-unit is configured to determine respective positions

of the common segmentation words in the symptom description information.

The first similarity determining sub-unit is configured to determine a first similarity between the segmentation word list and the symptom description information based on the number of common segmentation words in the segmentation word list and the number of segmentation words in the segmentation word list.

The first similarity determining sub-unit is further configured to determine the first similarity between the segmentation word list and the symptom description information based on a ratio of the number of common segmentation words in the segmentation word list to the number of segmentation words in the segmentation word list.

The measures of dispersion determining sub-unit is configured to determine a measures of dispersion between the HPO and the symptom description information based on the respective positions of the common segmentation words in the symptom description information and respective positions of the common segmentation words in the HPO.

Optionally, the measures of dispersion determining sub-unit is configured to: determine a first position vector of the segmentation word list in the symptom description information based on the respective positions of the common segmentation words in the symptom description information; determine a second position vector of the segmentation word list in the HPO based on the respective positions of the common segmentation words in the HPO; and determine the measures of dispersion between the HPO and the symptom description information based on a corresponding first position vector and a corresponding second position vector of each segmentation word list.

The position of the common segmentation word in the symptom description information can be a character serial number. It should be noted that the character serial number can be related to the number of characters in each segmentation word in the segmentation word list.

The first position vector may be a one dimensional list composed of the character serial numbers of the common segmentation words in the symptom description information.

The position of a common segmentation word in the HPO can be a character serial number. It should be noted that the character serial number can be related to the number of characters of each segmentation word in the segmentation word list.

The second position vector may be a one dimensional list consisting of the character serial numbers of the common segmentation words in the HPO.

Optionally, the measures of dispersion determining sub-unit is configured to determine a measures of dispersion between the HPO and the symptom description information by a following formula:

$$VAR = \sqrt{\frac{\sum_{n=1}^{N}\left(a_n - b_n - \frac{\sum_{n=1}^{N}(a_n - b_n)}{N}\right)^2}{N-1}}$$

where, *VAR* is the measures of dispersion between the HPO and the symptom description information, *N* is the number of segmentation word lists in the segmentation word group, $a_n$ is a first position vector of the segmentation word list *n* in the symptom description information, and $b_n$ is a second position vector of the segmentation word list *n* in the HPO.

The second similarity determining sub-unit is configured to determine a second similarity between the HPO and the symptom description information based on a corresponding first similarity of each segmentation word list.

Optionally, the second similarity determining sub-unit is configured to: determine a third similarity between the HPO and the symptom description information based on the corresponding first similarity of each segmentation word list; and determine the second similarity between the HPO and the symptom description information by multiplying the third similarity by an adjustable weighting factor.

Optionally, the second similarity determining sub-unit is further configured to: determine the third similarity between the HPO and the symptom description information by a following formula:

$$preScore = \sum_{n=1}^{N} \alpha\beta_n w_n \log p_n$$

where, *n* is a serial number of each segmentation word list, *preScore* is the third similarity, $\alpha\beta_n w_n \log p_n$ is the first similarity of the segmentation word list *n*, where $1 \leq n \leq N$, *N* is the number of segmentation word lists in the segmentation word group, $\alpha$ is an adjustment factor, $\beta_n$ is a normalization factor corresponding to the segmentation word list *n*, $w_n$ is a weight corresponding to the segmentation word list *n*, and $p_n$ is a ratio of the number of common segmentation words in the segmentation word list *n* to the number of segmentation words in the segmentation word list n.

The adjustable weighting factor can be set by the technician. The technicians can adjust the adjustable weighting

factor according to the execution result after each execution of the method for determining the HPO according to embodiments of the disclosure.

The normalization factor corresponding to the segmentation word list n can be expressed as:

$$\beta_n = \frac{logp_n - logp}{S^2}$$

where, $logp$ is an average value of data $logp_1, \ldots, logp_n$, which can be expressed as:

$$logp = \frac{\sum_{n=1}^{N} logp_n}{n}$$

where, $S^2$ is a variance of data $logp_1, \ldots, logp_n$, which can be expressed as:

$$S^2 = \frac{\sum_{n=1}^{N} (logp_n - logp)^2}{n}.$$

The weight corresponding to each segmentation word list can be set by the technicians. The technicians can adjust the weight according to the execution result after each execution of the method for determining the HPO according to embodiments of the disclosure.

The second similarity determining sub-unit is configured to determine the second similarity between the HPO and the symptom description information by a following formula:

$$Score = \gamma \times exp(preScore)$$

where, *Score* is the second similarity between the HPO and the symptom description information, $\gamma$ is the adjustable weighting factor.

It is noted that the adjustable weighting factor is related to the HPO. In detail, embodiments of the disclosure may determine the adjustable weighting factor based on historical matching data of the HPO. In detail, in embodiments of the disclosure, the historical matching data of the HPO can be extracted from the historical matching database. The historical matching data may include: a first number of times the HPO is correctly matched, a second number of times the HPO is incorrectly matched, and a third number of times the HPO is manually labeled. Embodiments of the disclosure may perform a weighted sum of the first number, the second number and the third number to determine the adjustable weighting factor. In embodiment of the disclosure, according to the historical matching data of the HPO, the adjustable weighting factor is determined and used to calculate the matching degree between the HPO and the symptom description information, which can improve the probability that the HPO is correctly matched.

The matching degree determining sub-unit is configured to determine a matching degree between the HPO and the symptom description information based on the second similarity, the measures of dispersion, and the number of segmentation word lists in the segmentation word group.

Optionally, the matching degree determining sub-unit is configured to determine the matching degree between the HPO and the symptom description information by a following formula:

$$AEEV = \frac{VAR}{N} \times Score$$

where, *AEEV* is the matching degree between the HPO and the symptom description information, *VAR* is the measures of dispersion between the HPO and the symptom description information, *Score* is the second similarity between the HPO and the symptom description information, and *N* is the number of segmentation word lists in the segmentation word group;

$$Score = \gamma \times exp(preScore)$$

where, $\gamma$ is the adjustable weighting factor, and *preScore* is the third similarity,

$$preScore=\sum_{n=1}^{N}\alpha\beta_n w_n \log p_n$$

where, $n$ is a serial number of each segmentation word list, $\alpha\beta_n w_n \log p_n$ is the first similarity of the segmentation word list $n$, where $1\leq n\leq N$, $\alpha$ is an adjustment factor, $\beta_n$ is a normalization factor corresponding to the segmentation word list $n$, $w_n$ is a weight corresponding to the segmentation word list $n$, and $p_n$ is a ratio of the number of common segmentation words in the segmentation word list $n$ to the number of segmentation words in the segmentation word list $n$;

$$VAR=\sqrt{\frac{\sum_{n=1}^{N}\left(a_n-b_n-\frac{\sum_{n=1}^{N}(a_n-b_n)}{N}\right)^2}{N-1}}$$

where, $a_n$ is a first position vector of the segmentation word list $n$ in the symptom description information, and $b_n$ is a second position vector of the segmentation word list $n$ in the HPO.

The HPO determining unit 300 is configured to determine an HPO, where the matching degree corresponding to the HPO satisfies a preset matching success condition.

The preset matching success condition may be the matching degree between the HPO and the symptom description information is greater than a preset matching degree threshold.

In embodiment of the disclosure, an HPO whose matching degree does not satisfy the preset matching success condition is filtered out, and only the HPO whose matching degree satisfies the preset matching success condition is output.

With an apparatus for determining a HPO according to embodiments of the disclosure, the symptom description information is obtained. The segmentation word group corresponding to an HPO in a preset HPO collection is queried and matched with the symptom description information, and the matching degree between the HPO and the symptom description information is determined. The segmentation word group includes at least one segmentation word list obtained by performing, according to a preset word segmentation rule, word segmentation on the HPO corresponding to the segmentation word group, and the segmentation word list includes at least one segmentation word. The HPO is determine such that, the matching degree corresponding to the HPO satisfies the preset matching success condition. In embodiments of the disclosure, querying and matching are performed between the symptom description information and segmentation word groups corresponding to various HPOs, and HPOs conforming to the symptom description information are screened out, the matching degree between the HPO and the symptom description information is determined, so as to determine the HPO that satisfies the symptom description information, thereby facilitating research and communication of the symptom description information by a technician, and facilitating data analysis and data mining of symptoms on the symptom description information.

Optionally, embodiments of the disclosure also provide another apparatus for determining a HPO. The apparatus includes: a description expression determining unit, a negative expression detecting unit, and a negative expression labeling unit.

The description expression determining unit is configured to determine, from the symptom description information, a description expression corresponding to the HPO, after the HPO determining unit 300 determines the HPO, in which the matching degree corresponding to the HPO satisfies the preset matching success condition.

In detail, in embodiments of the disclosure, according to the measures of dispersion of the common segmentation words in each segmentation word list corresponding to the HPO in the symptom description information, the expression at this position can be determined as the corresponding description expression of the HPO in the symptom description information.

The negative expression detecting unit is configured to detect whether an adjacent expression adjacent to the description expression is a preset negative expression, and trigger the negative expression labeling unit in response to detecting that the adjacent expression is the preset negative expression.

The negative expression labeling unit is configured to label the adjacent expression.

In embodiments of the disclosure, it is detected whether an adjacent expression adjacent to and prior to the description expression is a preset negative expression, and the adjacent expression is labeled with a preset negative identifier in response to detecting that the adjacent expression is the preset negative expression.

Optionally, embodiments of the disclosure provide another apparatus for determining a HPO, the apparatus

further includes: a description expression determining unit, an expression determining unit and an expression matching determining unit.

The description expression determining unit is configured to determine, from the symptom description information, a description expression corresponding to the HPO.

The expression determining unit is configured to determine whether the description expression is consistent with the HPO, trigger the expression matching determining unit in response to the description expression being consistent with the HPO, in which the expression matching determining unit is configured to determine that the HPO is completely matched with the description expression, and trigger the expression matching determining unit in response to the description expression being inconsistent with the HPO, in which the expression matching determining unit is configured to determine that the HPO is roughly matched with the description expression.

The apparatus for determining a HPO includes a processor and a memory. The above symptom description information obtaining unit 100, the matching degree determining unit 200, and the HPO determining unit 300 are all stored in the memory as program units. The processor executes the program units stored in the memory to realize the corresponding function.

The processor includes a core, and the core calls the corresponding program unit from the memory. There may be one or more cores. By adjusting the core parameters, the symptom description information can be matched with the corresponding segmentation word groups corresponding to the HPO, and the matching degree between the HPO and the symptom description information can be determined, so as to determine the HPO satisfying the symptom description information.

The embodiments of the disclosure provide a storage medium, having a program stored thereon. When the program is executed by a processor, a method for determining a HPO is executed.

The embodiments of the disclosure provide a processor, for running a program. When the program is running, a method for determining a HPO is executed.

The embodiments of the disclosure provide a device, including at least one processor, at least one memory and a bus, the at least one memory and the bus are connected to the processor. The processor communicates with the memory through the bus. The processor is configured to call program instructions in the memory to execute the above method for determining a HPO. The device in the disclosure can be a server, a PC, a PAD, and a mobile phone.

The embodiments of the disclosure provide a computer program product, suitable to a program for executing the method for determining a HPO when being executed on a data processing device.

The disclosure is described with reference to flowcharts and/or block diagrams of methods, devices (systems), and computer program products according to the embodiments of the disclosure. It should be understood that each process and/or block of the flowchart and/or block diagram, and combinations of processes and/or blocks of the flowchart and/or block diagram can be executed by computer program instructions. The computer program instructions can be provided to the processor of a general-purpose computer, a special computer, an embedded processor, or other programmable data processing device to generate a machine, so that the instructions executed by the processor of the computer or other programmable data processing device are used to implement the functions specified in one process or multiple processes of the flowchart and/or one block or multiple blocks of the block diagram.

In a typical configuration, the device includes one or more processors (CPUs), memories, and buses. The device may also include input/output interfaces, network interfaces, and so on.

The memory may include non-permanent memory in a computer-readable medium, random access memory (RAM) and/or non-volatile memory, such as read-only memory (ROM) or flash memory (flashRAM). The memory includes at least one memory chip. The memory is an example of a computer-readable medium.

Computer-readable mediums include permanent medium, non-permanent medium, removable medium and non-removable medium, configured to store information by any method or technology. The information may be computer-readable instructions, data structures, program modules, or other data. Examples of computer storage mediums include, but are not limited to, phase change memory (PRAM), static random access memory (SRAM), dynamic random access memory (DRAM), other types of random access memory (RAM), read-only memory (ROM), electrically erasable programmable read-only memory (EEPROM), flash memory or other optical storage, magnetic cassettes, magnetic tape or magnetic disk or other magnetic storage devices or any other non-transmission medium that can be used to store information that can be accessed by computing devices. According to the definition in the disclosure, computer-readable medium does not include transitory media, such as modulated data signal and carrier.

It should be noted that the terms "include", "comprise", or any other variants thereof are intended to cover non-exclusive inclusion, so that a process, method, article or device that includes a series of elements includes not only those elements, but also other elements that are not explicitly listed, or also include elements inherent to such process, method, article or device. Without more restrictions, the element defined by the sentence "comprising a..." does not exclude the existence of other identical elements in the process, method, article or device that includes the element.

Those skilled in the art should understand that the embodiments of the disclosure can be provided as a method, a system, or a computer program product. Therefore, the disclosure may adopt the form of a complete hardware embodiment, a complete software embodiment, or an embodiment combining software and hardware. Moreover, the disclosure may adopt the form of a computer program product implemented on one or more computer-usable storage media (including but not limited to disk storage, CD-ROM and optical storage) containing computer-usable program codes.

The above are only examples of the disclosure, and are not used to limit the disclosure. For those skilled in the art, the disclosure can have various modifications and changes. Any modification, equivalent replacement and improvement made within the spirit and principle of the disclosure shall be included in the scope of the claims of the disclosure.

## Claims

1. A method for determining a Human Phenotype Ontology, HPO, comprising:

    obtaining symptom description information;
    for a segmentation word list in a segmentation word group corresponding to an HPO in a preset HPO collection, querying, from the symptom description information, a segmentation word of the segmentation word list, determining a segmentation word included in both the segmentation word list and the symptom description information as a common segmentation word; determining the number of common segmentation words in the segmentation word list and respective positions of the common segmentation words in the symptom description information;
    determining a first similarity between the segmentation word list and the symptom description information based on the number of common segmentation words in the segmentation word list and the number of segmentation words in the segmentation word list; determining a measures of dispersion between the HPO and the symptom description information based on the respective positions of the common segmentation words in the symptom description information and respective positions of the common segmentation words in the HPO;
    determining a second similarity between the HPO and the symptom description information based on a corresponding first similarity of each segmentation word list;
    determining a matching degree between the HPO and the symptom description information based on the second similarity, the measures of dispersion, and the number of segmentation word lists in the segmentation word group; and
    determining an HPO, wherein the matching degree corresponding to the HPO satisfies a preset matching success condition.

2. The method according to claim 1, wherein segmentation words are different between each segmentation word list of the segmentation word group, and the number of words of each segmentation word is the same for the same segmentation word list.

3. The method according to claim 1, wherein after determining the HPO, in which the matching degree corresponding to the HPO satisfies the preset matching success condition, the method further comprises:

    determining, from the symptom description information, a description expression corresponding to the HPO; and
    detecting whether an adjacent expression adjacent to the description expression is a preset negative expression, and labeling the adjacent expression in response to determining that the adjacent expression is the preset negative expression.

4. The method according to claim 1, wherein after determining the HPO, in which the matching degree corresponding to the HPO satisfies the preset matching success condition, the method further comprises:

    determining, from the symptom description information, a description expression corresponding to the HPO; and
    determining whether the description expression is consistent with the HPO,
    determining that the HPO is completely matched with the description expression, in response to the description expression being consistent with the HPO, and
    determining that the HPO is roughly matched with the description expression, in response to the description expression being inconsistent with the HPO.

5. The method according to claim 1, wherein determining the second similarity between the HPO and the symptom description information based on the corresponding first similarity of each segmentation word list comprises:

    determining a third similarity between the HPO and the symptom description information based on the corresponding first similarity of each segmentation word list; and
determining the second similarity between the HPO and the symptom description information by multiplying the third similarity by an adjustable weighting factor.

6. The method according to claim 1, wherein determining the measures of dispersion between the HPO and the symptom description information based on respective positions of the common segmentation words in the symptom description information and the respective positions of the common segmentation words in the HPO, comprises:

determining a first position vector of the segmentation word list in the symptom description information based on the respective positions of the common segmentation words in the symptom description information;
determining a second position vector of the segmentation word list in the HPO based on the respective positions of the common segmentation words in the HPO; and
determining the measures of dispersion between the HPO and the symptom description information based on a corresponding first position vector and a corresponding second position vector of each segmentation word list.

7. The method according to claim 1, wherein determining the matching degree between the HPO and the symptom description information based on the second similarity, the measures of dispersion, and the number of segmentation word lists in the segmentation word group, comprises:

$$AEEV=\frac{VAR}{N}\times Score$$

where, *AEEV* is the matching degree between the HPO and the symptom description information, *VAR* is the measures of dispersion between the HPO and the symptom description information, and *Score* is the second similarity between the HPO and the symptom description information, and *N* is the number of segmentation word lists in the segmentation word group;

$$Score=\gamma\times exp(preScore)$$

where, $\gamma$ is an adjustable weighting factor, and *preScore* is a third similarity,

$$preScore=\sum_{n=1}^{N}\alpha\beta_n w_n\, logp_n$$

where, $n$ is a serial number of each segmentation word list, $\alpha\beta_n w_n logp_n$ is the first similarity of the segmentation word list $n$, where $1\leq n\leq N$, $\alpha$ is an adjustment factor, $\beta_n$ is a normalization factor corresponding to the segmentation word list $n$, $w_n$ is a weight corresponding to the segmentation word list $n$, and $p_n$ is a ratio of the number of common segmentation words in the segmentation word list $n$ to the number of segmentation words in the segmentation word list $n$;

$$VAR=\sqrt{\frac{\sum_{n=1}^{N}\left(a_n-b_n-\frac{\sum_{n=1}^{N}(a_n-b_n)}{N}\right)^2}{N-1}}$$

where, a, is a first position vector of the segmentation word list $n$ in the symptom description information, and $b_n$ is a second position vector of the segmentation word list $n$ in the HPO.

8. An apparatus for determining a Human Phenotype Ontology (HPO), comprising: a symptom description information obtaining unit, a matching degree determining unit, and a HPO determining unit, wherein the matching degree determining unit comprises: a common segmentation word determining sub-unit, a common segmentation word number determining sub-unit, a common segmentation word position determining sub-unit, a first similarity determining sub-unit, a measures of dispersion determining sub-unit, a second similarity determining sub-unit, and a

matching degree determining sub-unit;

the symptom description information obtaining unit is configured to obtain symptom description information;
the common segmentation word determining sub-unit is configured to, for a segmentation word list in a segmentation word group corresponding to an HPO in a preset HPO collection, query, from the symptom description information, a segmentation word of the segmentation word list, and determine a segmentation word included in both the segmentation word list and the symptom description information as a common segmentation word;
the common segmentation word number determining sub-unit is configured to determine the number of common segmentation words in the segmentation word list;
the common segmentation word position determining sub-unit is configured to determine respective positions of the common segmentation words in the symptom description information;
the first similarity determining sub-unit is configured to determine a first similarity between the segmentation word list and the symptom description information based on the number of common segmentation words in the segmentation word list and the number of segmentation words in the segmentation word list;
the measures of dispersion determining sub-unit is configured to determine a measures of dispersion between the HPO and the symptom description information based on the respective positions of the common segmentation words in the symptom description information and respective positions of the common segmentation words in the HPO;
the second similarity determining sub-unit is configured to determine a second similarity between the HPO and the symptom description information based on a corresponding first similarity of each segmentation word list;
the matching degree determining sub-unit is configured to determine a matching degree between the HPO and the symptom description information based on the second similarity, the measures of dispersion, and the number of segmentation word lists in the segmentation word group; and
the HPO determining unit is configured to determine an HPO, wherein the matching degree corresponding to the HPO satisfies a preset matching success condition.

**9.** The apparatus according to claim 8, comprising: a description expression determining unit, a negative expression detecting unit, and a negative expression labeling unit, wherein,

the description expression determining unit is configured to determine, from the symptom description information, a description expression corresponding to the HPO, after determining the HPO, in which the matching degree corresponding to the HPO satisfies the preset matching success condition;
the negative expression detecting unit is configured to detect whether an adjacent expression adjacent to the description expression is a preset negative expression, and trigger the negative expression labeling unit in response to detecting that the adjacent expression is the preset negative expression; and
the negative expression labeling unit is configured to label the adjacent expression.

**10.** The apparatus according to claim 8, comprising: a description expression determining unit, an expression determining unit and an expression matching determining unit, wherein,

the description expression determining unit is configured to determine, from the symptom description information, a description expression corresponding to the HPO; and
the expression determining unit is configured to determine whether the description expression is consistent with the HPO, trigger the expression matching determining unit in response to the description expression being consistent with the HPO, wherein the expression matching determining unit is configured to determine that the HPO is completely matched with the description expression, and trigger the expression matching determining unit in response to the description expression being inconsistent with the HPO, wherein the expression matching determining unit is configured to determine that the HPO is roughly matched with the description expression.

**11.** A storage medium, having a program stored thereon, wherein when the program is executed by a processor, a method for determining a HPO according to any one of claims 1 to 7 is executed.

**12.** A processor, for running a program, wherein when the program is running, a method for determining a HPO according to any one of claims 1 to 7 is executed.

**13.** A device, comprising at least one processor, at least one memory and a bus, wherein the at least one memory and the bus are connected to the processor, the processor communicates with the memory through the bus; the processor is configured to call program instructions in the memory to execute a method for determining a HPO according to

any one of claims 1 to 7.

**14.** A computer program product, suitable to a program for executing a method for determining a HPO according to any one of claims 1 to 7 when being executed on a data processing device.

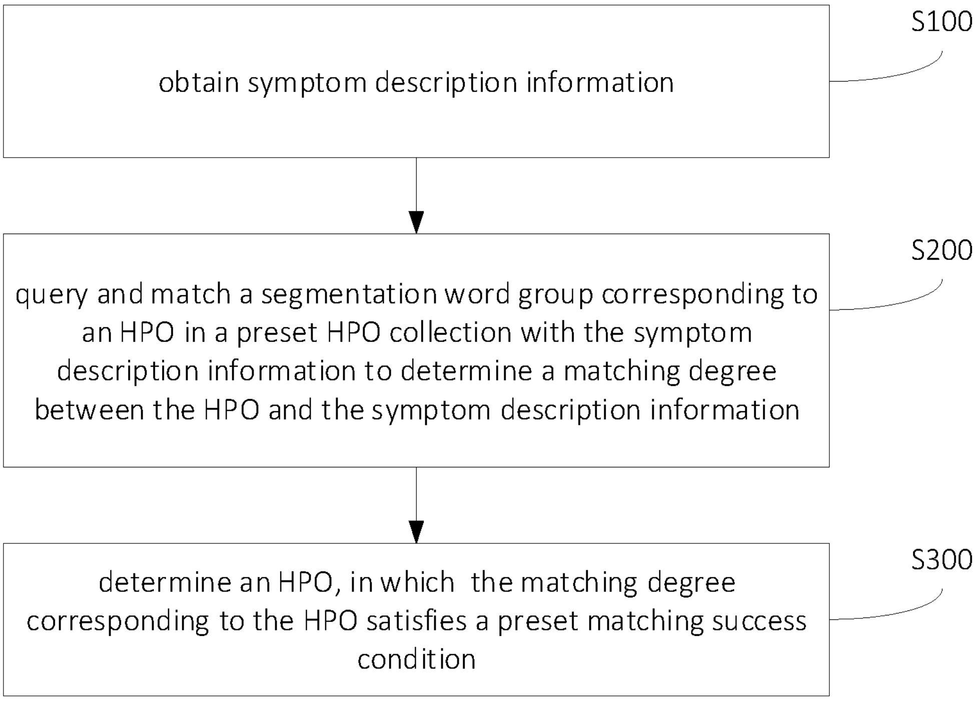
S100
obtain symptom description information
S200
query and match a segmentation word group corresponding to an HPO in a preset HPO collection with the symptom description information to determine a matching degree between the HPO and the symptom description information
S300
determine an HPO, in which the matching degree corresponding to the HPO satisfies a preset matching success condition

FIG. 1

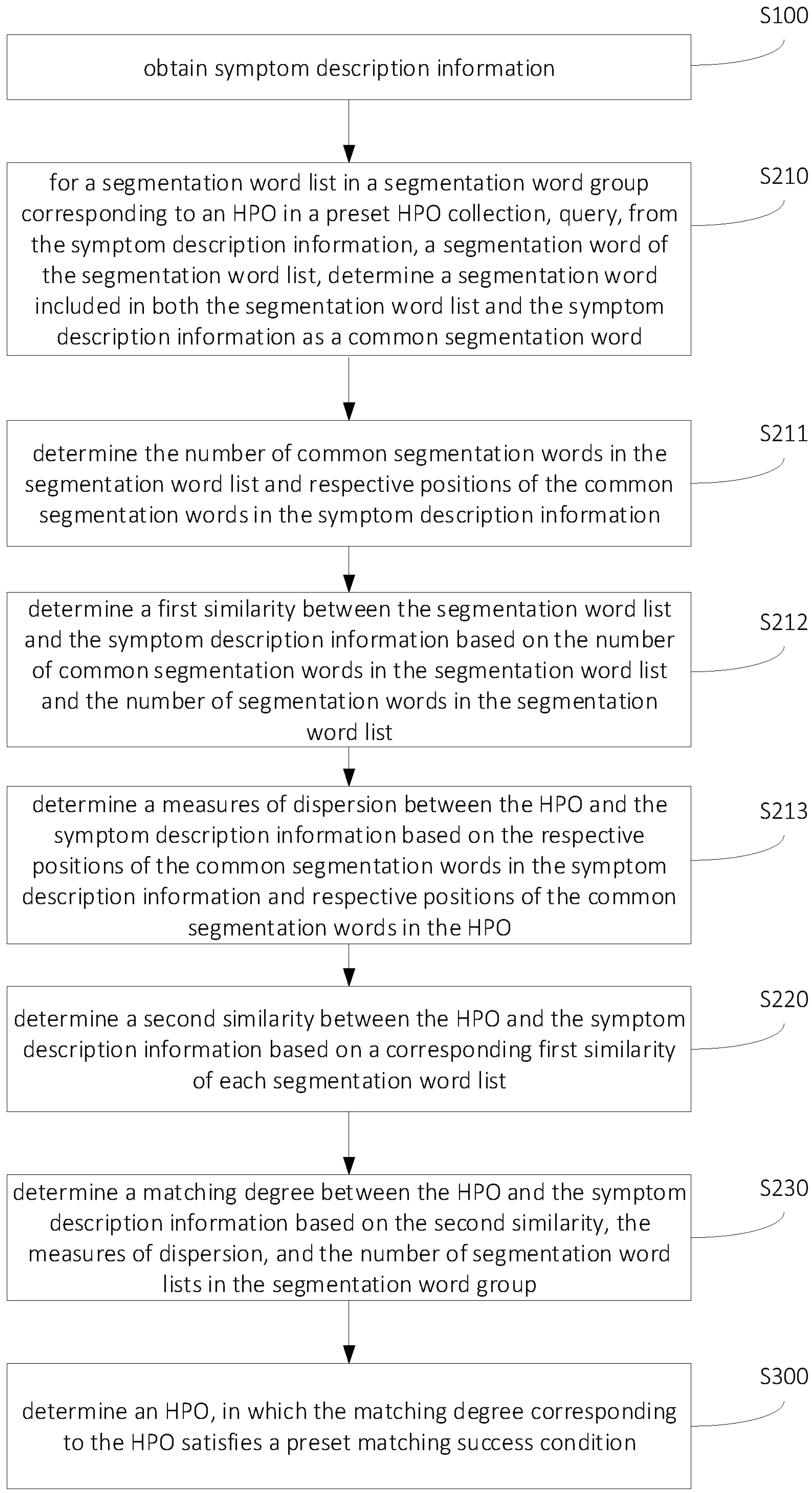
S100
obtain symptom description information
S210
for a segmentation word list in a segmentation word group corresponding to an HPO in a preset HPO collection, query, from the symptom description information, a segmentation word of the segmentation word list, determine a segmentation word included in both the segmentation word list and the symptom description information as a common segmentation word
S211
determine the number of common segmentation words in the segmentation word list and respective positions of the common segmentation words in the symptom description information
S212
determine a first similarity between the segmentation word list and the symptom description information based on the number of common segmentation words in the segmentation word list and the number of segmentation words in the segmentation word list
S213
determine a measures of dispersion between the HPO and the symptom description information based on the respective positions of the common segmentation words in the symptom description information and respective positions of the common segmentation words in the HPO
S220
determine a second similarity between the HPO and the symptom description information based on a corresponding first similarity of each segmentation word list
S230
determine a matching degree between the HPO and the symptom description information based on the second similarity, the measures of dispersion, and the number of segmentation word lists in the segmentation word group
S300
determine an HPO, in which the matching degree corresponding to the HPO satisfies a preset matching success condition

FIG. 2

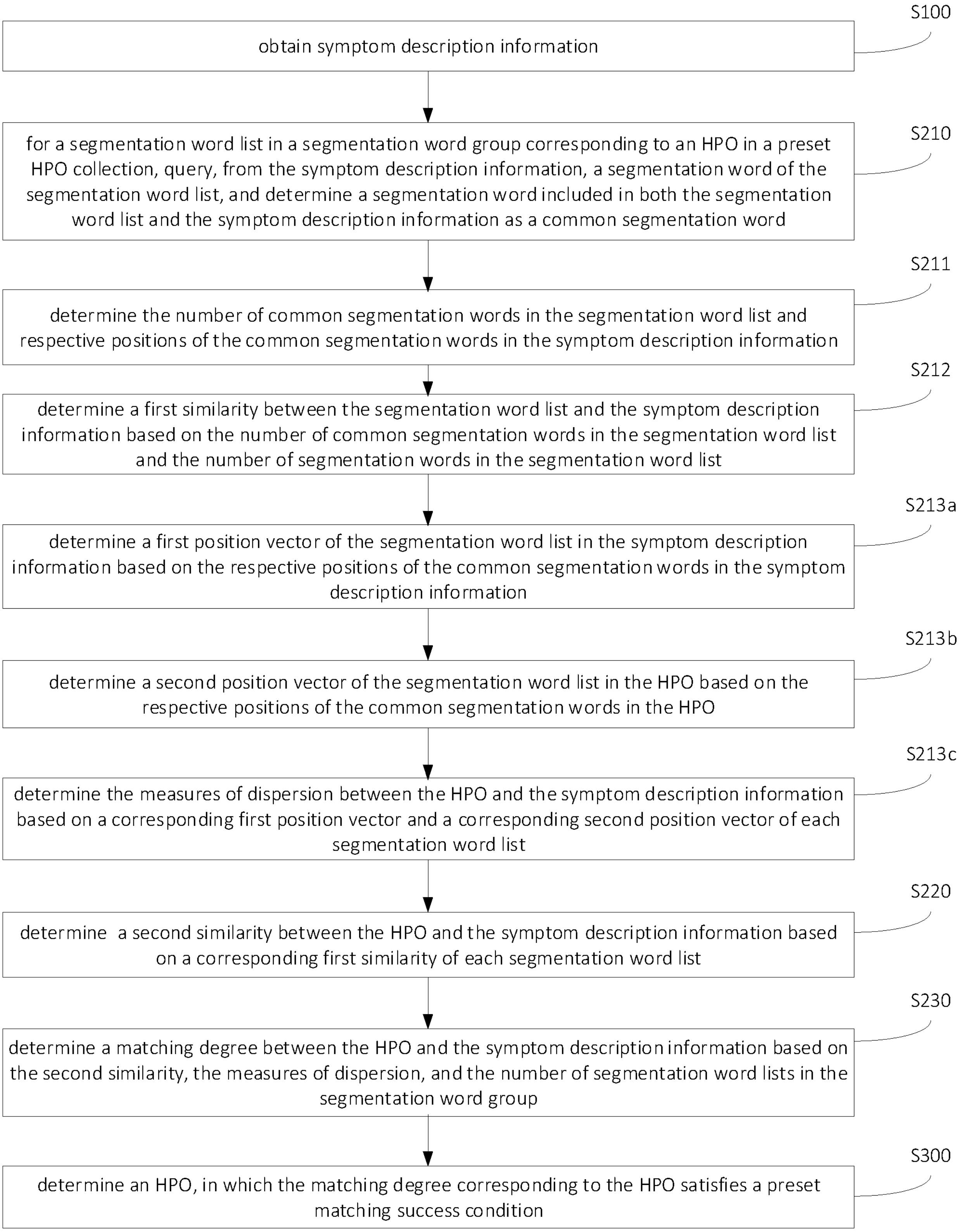
S100
obtain symptom description information
S210
for a segmentation word list in a segmentation word group corresponding to an HPO in a preset HPO collection, query, from the symptom description information, a segmentation word of the segmentation word list, and determine a segmentation word included in both the segmentation word list and the symptom description information as a common segmentation word
S211
determine the number of common segmentation words in the segmentation word list and respective positions of the common segmentation words in the symptom description information
S212
determine a first similarity between the segmentation word list and the symptom description information based on the number of common segmentation words in the segmentation word list and the number of segmentation words in the segmentation word list
S213a
determine a first position vector of the segmentation word list in the symptom description information based on the respective positions of the common segmentation words in the symptom description information
S213b
determine a second position vector of the segmentation word list in the HPO based on the respective positions of the common segmentation words in the HPO
S213c
determine the measures of dispersion between the HPO and the symptom description information based on a corresponding first position vector and a corresponding second position vector of each segmentation word list
S220
determine a second similarity between the HPO and the symptom description information based on a corresponding first similarity of each segmentation word list
S230
determine a matching degree between the HPO and the symptom description information based on the second similarity, the measures of dispersion, and the number of segmentation word lists in the segmentation word group
S300
determine an HPO, in which the matching degree corresponding to the HPO satisfies a preset matching success condition

FIG. 3

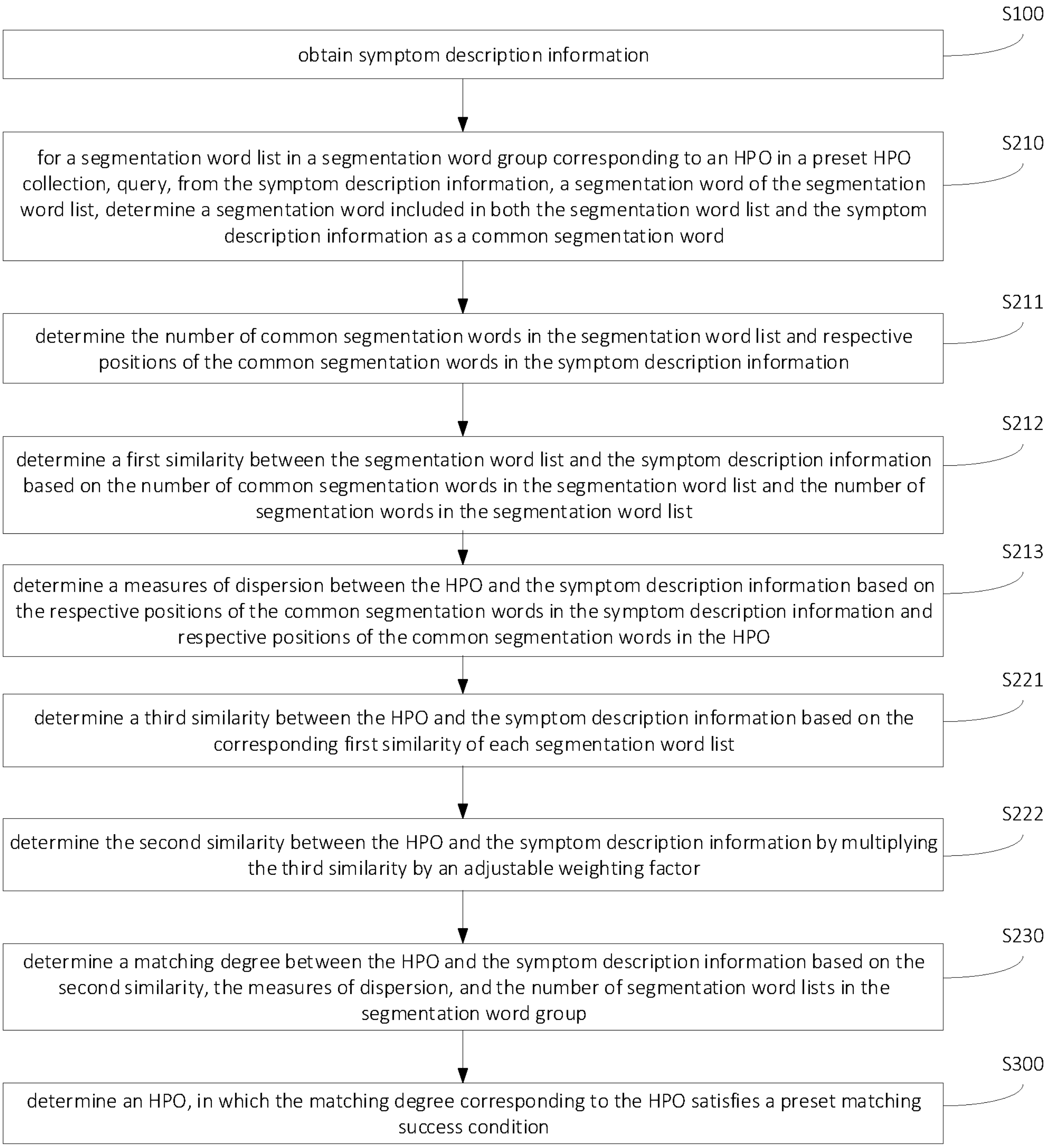
S100
obtain symptom description information
S210
for a segmentation word list in a segmentation word group corresponding to an HPO in a preset HPO collection, query, from the symptom description information, a segmentation word of the segmentation word list, determine a segmentation word included in both the segmentation word list and the symptom description information as a common segmentation word
S211
determine the number of common segmentation words in the segmentation word list and respective positions of the common segmentation words in the symptom description information
S212
determine a first similarity between the segmentation word list and the symptom description information based on the number of common segmentation words in the segmentation word list and the number of segmentation words in the segmentation word list
S213
determine a measures of dispersion between the HPO and the symptom description information based on the respective positions of the common segmentation words in the symptom description information and respective positions of the common segmentation words in the HPO
S221
determine a third similarity between the HPO and the symptom description information based on the corresponding first similarity of each segmentation word list
S222
determine the second similarity between the HPO and the symptom description information by multiplying the third similarity by an adjustable weighting factor
S230
determine a matching degree between the HPO and the symptom description information based on the second similarity, the measures of dispersion, and the number of segmentation word lists in the segmentation word group
S300
determine an HPO, in which the matching degree corresponding to the HPO satisfies a preset matching success condition

FIG. 4

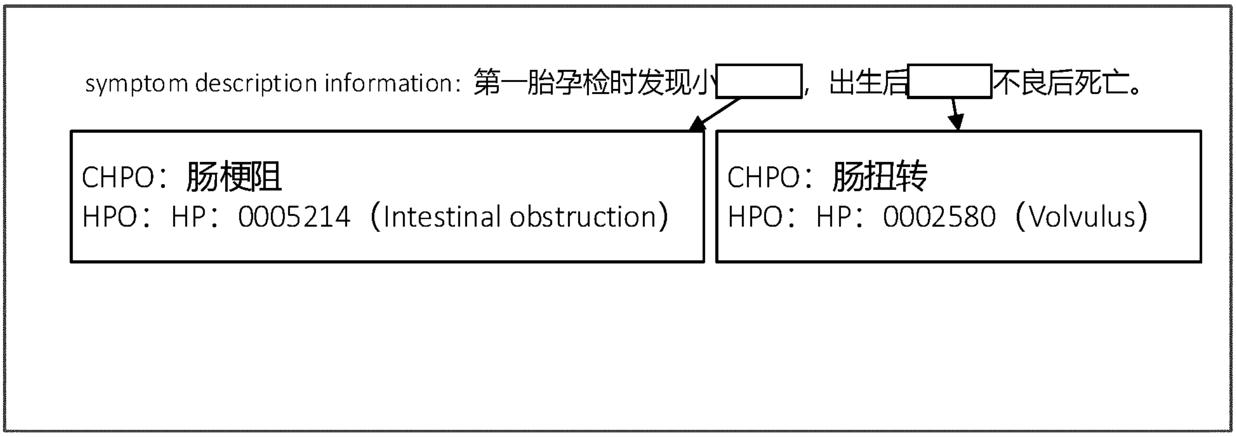
symptom description information：第一胎孕检时发现小
，出生后
不良后死亡。
CHPO：肠梗阻
HPO：HP：0005214（Intestinal obstruction）
CHPO：肠扭转
HPO：HP：0002580（Volvulus）

FIG. 5

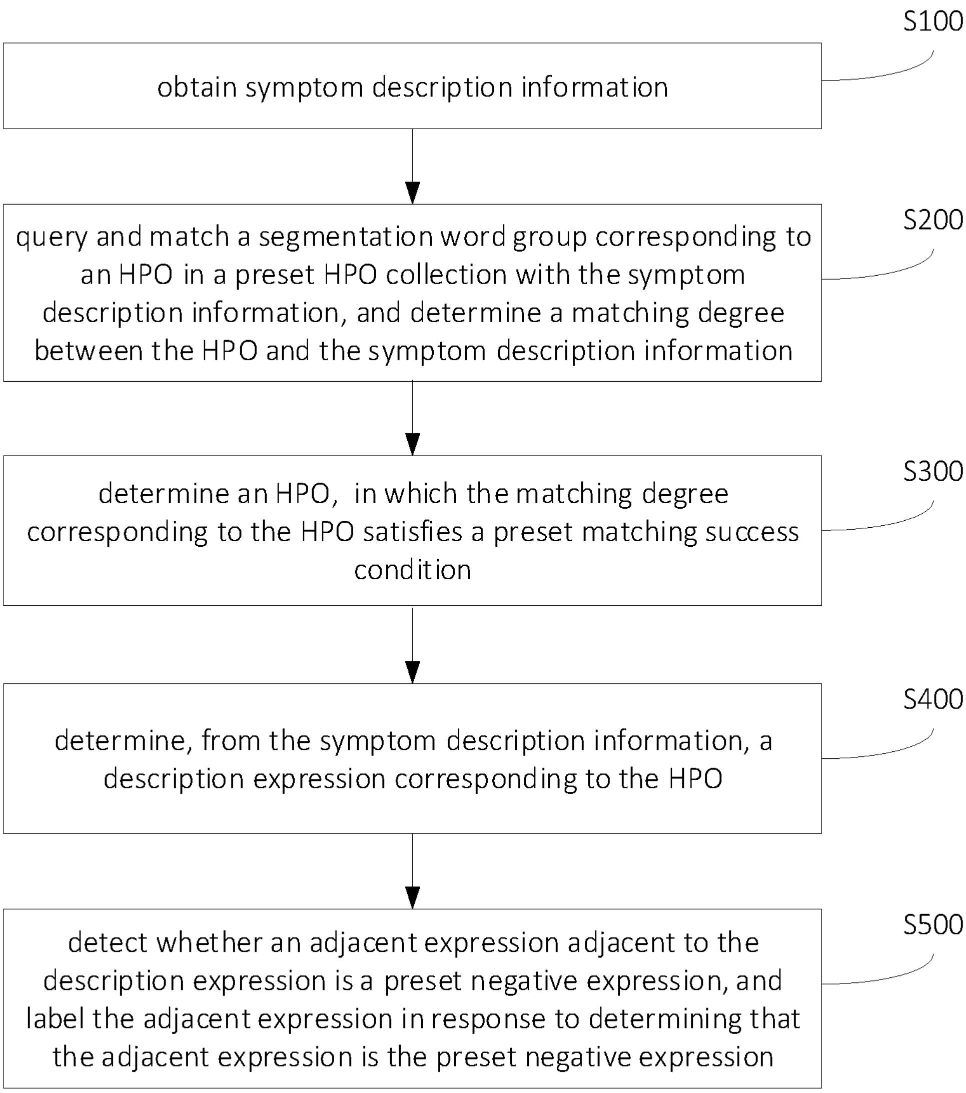
S100
obtain symptom description information
S200
query and match a segmentation word group corresponding to an HPO in a preset HPO collection with the symptom description information, and determine a matching degree between the HPO and the symptom description information
S300
determine an HPO, in which the matching degree corresponding to the HPO satisfies a preset matching success condition
S400
determine, from the symptom description information, a description expression corresponding to the HPO
S500
detect whether an adjacent expression adjacent to the description expression is a preset negative expression, and label the adjacent expression in response to determining that the adjacent expression is the preset negative expression

FIG. 6

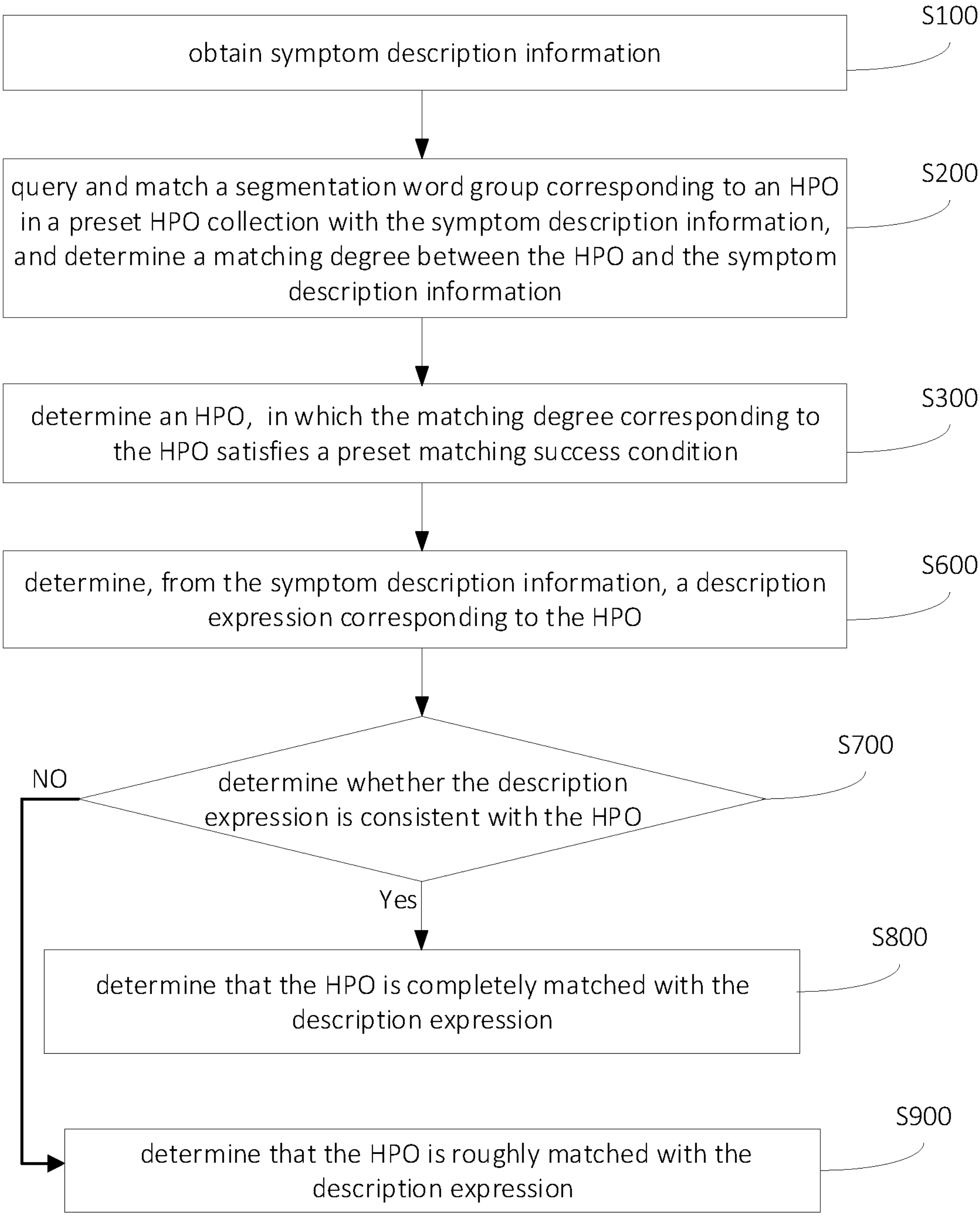
S100
obtain symptom description information
S200
query and match a segmentation word group corresponding to an HPO in a preset HPO collection with the symptom description information, and determine a matching degree between the HPO and the symptom description information
S300
determine an HPO, in which the matching degree corresponding to the HPO satisfies a preset matching success condition
S600
determine, from the symptom description information, a description expression corresponding to the HPO
S700
determine whether the description expression is consistent with the HPO
NO
Yes
S800
determine that the HPO is completely matched with the description expression
S900
determine that the HPO is roughly matched with the description expression

FIG. 7

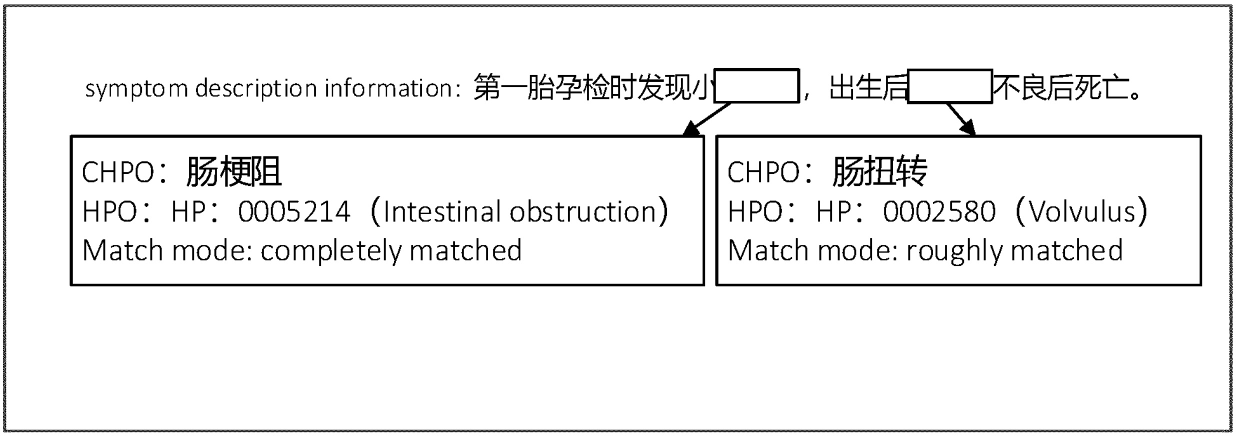
symptom description information：第一胎孕检时发现小 ，出生后 不良后死亡。
CHPO：肠梗阻
HPO：HP：0005214（Intestinal obstruction）
Match mode: completely matched
CHPO：肠扭转
HPO：HP：0002580（Volvulus）
Match mode: roughly matched

FIG. 8

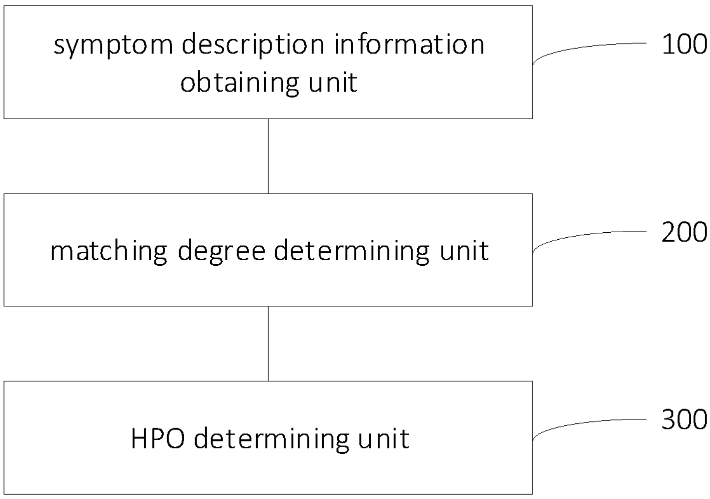
symptom description information obtaining unit
100
matching degree determining unit
200
HPO determining unit
300

FIG. 9

## INTERNATIONAL SEARCH REPORT

International application No.
**PCT/CN2020/127048**

**A. CLASSIFICATION OF SUBJECT MATTER**

G06F 40/117(2020.01)i

According to International Patent Classification (IPC) or to both national classification and IPC

**B. FIELDS SEARCHED**

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNKI, CNPAT, WPI, EPODOC, IEEE: 症状, 分词, 相似度, 数量, 位置, 匹配, symptom, word, segment, similar, number, location, match

**C. DOCUMENTS CONSIDERED TO BE RELEVANT**

| Category* | Citation of document, with indication, where appropriate, of the relevant passages | Relevant to claim No. |
|---|---|---|
| PX | CN 111159977 A (BGI TECH SOLUTIONS (SHENZHEN) CO., LTD.) 15 May 2020 (2020-05-15)<br>claims 1-10, description paragraphs [0106]-[0109] | 1-14 |
| A | CN 109753555 A (PING AN TECHNOLOGY (SHENZHEN) CO., LTD.) 14 May 2019 (2019-05-14)<br>description, paragraphs [0052]-[0092] | 1-14 |
| A | CN 109949927 A (SICHUAN SHIZHI LIANXING TECHNOLOGY CO., LTD.) 28 June 2019 (2019-06-28)<br>entire document | 1-14 |
| A | CN 109473169 A (AEGICARE (SHENZHEN) TECHNOLOGY CO., LTD.) 15 March 2019 (2019-03-15)<br>entire document | 1-14 |
| A | CN 110335684 A (UNIVERSITY OF ELECTRONIC SCIENCE AND TECHNOLOGY OF CHINA) 15 October 2019 (2019-10-15)<br>entire document | 1-14 |
| A | US 2006085590 A1 (3COM CORPORATION) 20 April 2006 (2006-04-20)<br>entire document | 1-14 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 January 2021** | **27 January 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/127048**

| Patent document cited in search report | | | Publication date (day/month/year) | Patent family member(s) | | | Publication date (day/month/year) |
|---|---|---|---|---|---|---|---|
| CN | 111159977 | A | 15 May 2020 | None | | | |
| CN | 109753555 | A | 14 May 2019 | None | | | |
| CN | 109949927 | A | 28 June 2019 | None | | | |
| CN | 109473169 | A | 15 March 2019 | None | | | |
| CN | 110335684 | A | 15 October 2019 | None | | | |
| US | 2006085590 | A1 | 20 April 2006 | GB | 2419197 | A | 19 April 2006 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010265146